# EUROPÄISCHE PATENTSCHRIFT

(11) **EP 1 090 648 B1**
(45) Veröffentlichungstag und Bekanntmachung des Hinweises auf die Patenterteilung: **14.07.2004**
(21) Anmeldenummer: 00121617.5
(22) Anmeldetag: 02.10.2000
(51) Int. Cl.: A61L 15/36

(54) **Tampon zur Anwendung in Körperhöhlen**
Tampon for use in body cavities
Tampon à utilisation dans les cavités corporelles

(30) Priorität: 07.10.1999 DE 19948419
(43) Veröffentlichungstag der Anmeldung: 11.04.2001
(73) Patentinhaber: Symbio Herborn Group GmbH & Co., 35745 Herborn-Hörbach (DE)
(72) Erfinder: Rusch, Volker, Dr., 35745 Herborn (DE); Solfronk, Joachim Bruno, 61350 Bad Homburg (DE); Eilbert, Peter, 35745 Herborn (DE); Brunsmann, Holger, 35689 Dillenburg (DE); Ganss, Christoph, Dr., 09599 Freiberg (DE)
(74) Vertreter: Pätzold, Herbert, Dr.

(56) Entgegenhaltungen:
- WO-A-92/13577
- WO-A-97/29763
- WO-A-98/44884
- WO-A-99/17813
- GB-A- 2 310 375

## Beschreibung

Die Erfindung betrifft einen Tampon gemäß dem Oberbegriff des Anspruches 1.

Es sind Tampons der vorstehenden Art, insbesondere Vaginaltampons bekannt, die während der Menstruation benutzt werden. Der Vaginaltampon besteht vielfach aus einem länglichen Wattebausch, der von einem dünnen vliesartigen Stoff ummantelt ist. Der Wattebausch ist mit dem vliesartigen Mantel in eine stäbchenförmige Gestalt mit radialen Kerben gepreßt. Dabei besitzt die Watte eine weitgehend hohe Saugkraft und vermag eine beachtliche Menge an Körperflüssigkeit aufzunehmen, wobei der Wattebausch radial aufquillt und der vliesartige Mantel sich dabei entfaltet, indem er sich aus den radialen Kerben löst.

Der vorstehende Vaginaltampon wird aus einem ersten Abschnitt einer eng querplisierten Wattebahn und einem zweiten Abschnitt einer dünnen vliesartigen Stoffbahn hergestellt. Der Wattebahnabschnitt wird über seine halbe Länge derart gefaltet, daß die beiden äußeren Bahnenden übereinander zu liegen kommen. Anschließend wird der hälftig gefaltete Bahnabschnitt aufgerollt, anschließend mit dem vliesartigen Stoffabschnitt ummantelt und sodann durch radiales Verpressen in seine anwendungsbreite Form hoch verdichtet.

Aufgabe der Erfindung ist es einen Tampon der vorstehenden Art ohne wesentliche Änderung seiner Grundstruktur und der Herstellungsweise derart weiterzubilden, daß er zur Symbiontentherapie insbesondere im Vaginalbereich außerhalb der Menstruationsphase geeignet ist. Hierdurch sollen Erkrankungen im Vaginalbereich vorgebeugt oder behandelt werden. Vergleichbare Tampons in entsprechend angepaßter Form sollen auch zur Symbiontentherapie in anderen Körperhöhlen, z.B. im Mund zur Therapie von Entzündungen im Mund- und Zahnfleischbereich, geeignet sein.

Die Aufgabe wird erfindungsgemäß mit den kennzeichnenden Merkmalen des Anspruches 1 gelöst.

Vorteilhafte Ausführungen können den Merkmalen der Unteransprüche entnommen werden.

Die Erfindung wird ohne jede Beschränkung anhand zweier Ausführungsbeispiele beschrieben, die in einer Zeichnung schematisch dargestellt ist. Hierin zeigt:
- Fig. 1: einen eng plissierten hydrophoben Wattebahnabschnitt in der Draufsicht,
- Fig. 2: den Wattebahnabschnitt zur Hälfte gefaltet,
- Fig. 3: den zur Hälfte gefalteten Wattebahnabschnitt in aufgerollter Form in perspektivischer Darstellung,
- Fig. 4: einen Abschnitt eines dünnen Vliesstoffes zur Ummantelung des lose aufgerollten Wattebahnabschnittes,
- Fig. 5: den dünnen Vliestoffabschnitt mit aufgebrachtem Bakterienlyophilisat nebst zugehörigem Nährmedium;
- Fig. 6: den aufgerollten Wattebahnabschnitt nach Fig. 3 ummantelt mit dem Vliesstoffabschnitt, der innenseitig das Lyophilisat trägt,
- Fig. 7: den zur Benutzung fertigen Tampon durch radiales Verpressen des unverpressten ummantelten Tampons nach Fig. 6,
- Fig. 8: einen verdoppelten Vliesstoffabschnitt und
- Fig. 9: den nach Fig. 8 längsgefalteten Vliesstoffabschnitt mit eingeschlossenem Bakterienlyophilisat.

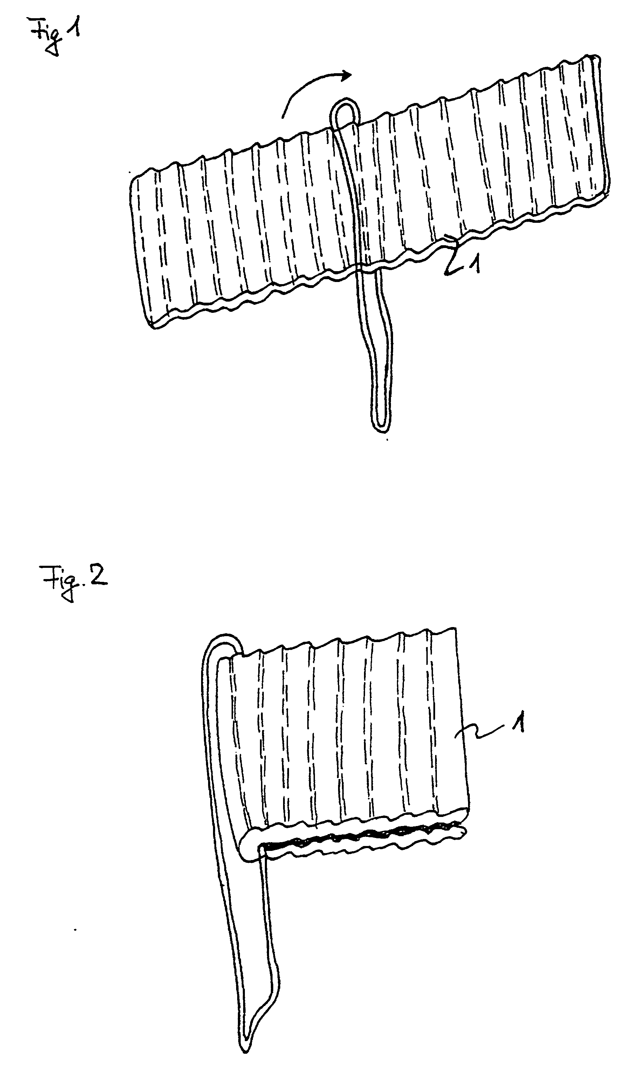

Der in an sich bekannter Weise eng querplissierte Wattebahnabschnitt 1 nach Fig. 1 besteht, anders als für herkömmliche Tampons, aus einem hydrophoben oder weitgehend hydrophoben Material, das keine oder nur geringe Mengen an Flüssigkeit aufnimmt und in einem feuchten Milieu praktisch keine Saugkraft entwickelt.

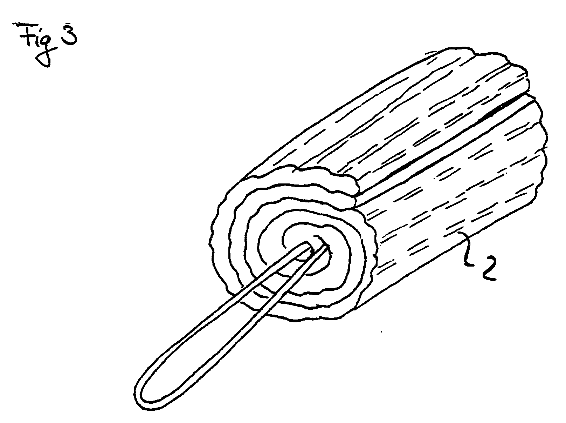

Wie Fig. 2 und 3 zeigt, wird der hydrophobe oder weitgehend hydrophobe Wattebahnabschnitt 1 in herkömmlicher Weise zur Hälfte gefaltet (Fig. 2) und sodann zu einem losen Tampon 2 aufgerollt.

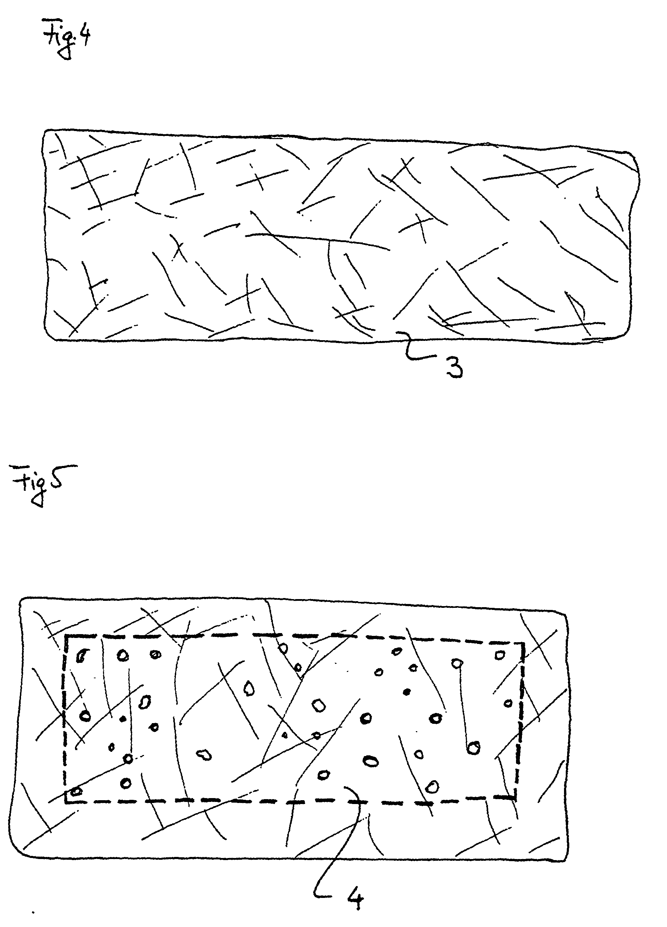

Fig. 4 zeigt einen dünnen Vliesstoffabschnitt 3 zur herkömmlichen Ummantelung des lose aufgerollten Tampons nach Fig. 3. Der Vliesstoffabschnitt 3 dient erfindungsgemäß nicht nur zur herkömmlichen Ummantelung des Tampons sondern auch als Träger von vorzugsweise lyophilisierten Bakterien 4 (Fig. 5), z.B. Milchsäure bildenden Bakterien zur Pflege oder zum Aufbau der Vaginal- und Scheidenflora. Die lyophilisierten Bakterien 4 in pulvriger oder globuliner Gestalt sind mit geeigneten Nährmedien versehen.

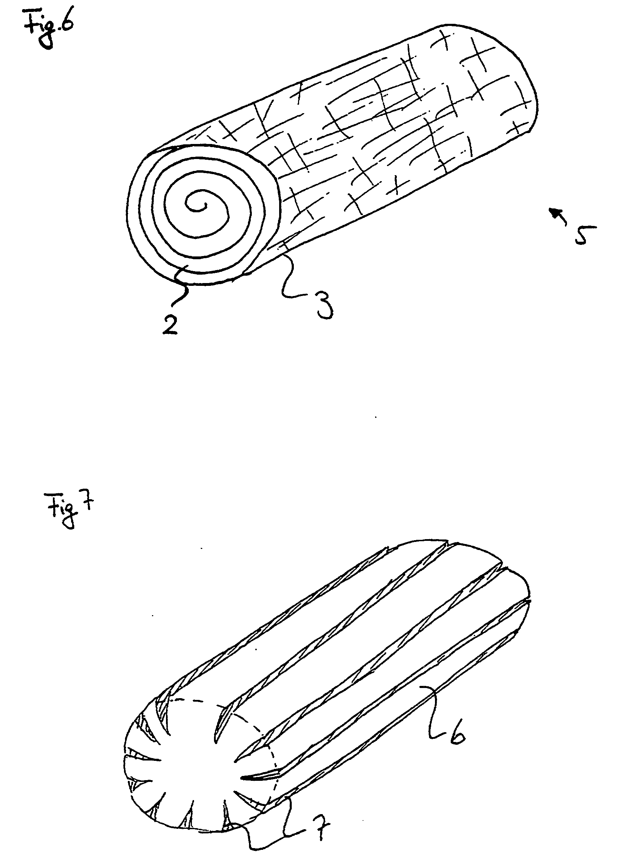

Der gemäß Fig. 3 locker aufgerollte Wattebahnabschnitt 2 wird in herkömmlicher Weise mit dem Vliesstoffabschnitt 3 ummantelt (Fig. 6), der innenseitig das Lyophilisat 4 mit dem Nährmedium trägt. Anschließend wird der locker aufgerollte und ummantelte Tampon 5 in herkömmlicher Weise in die benutzungsbereite Form 6 (Fig. 7) radial verdichtet, wobei längs verlaufende Rillen 7 gebildet werden, in denen der Vliesstoff eingefaltet ist.

Der in seine zur Benutzung fertige Form hoch verdichtete Tampon 6 wird in einem feuchten Milieu in einer Körperhöhle, z.B. der Vagina, nur oberflächlich benetzt, wodurch die lyophilisierten Bakterien zwischen dem vliesartigen Mantel und der weitgehend hydrophoben Watte aktiviert werden. Die Bakterien können dank des fehlenden Saugdruckes des erfindungsgemäßen Tampons durch den vliesartigen Mantel leicht hindurchtreten und z.B. innerhalb einer Scheide das Scheidenepithel besiedeln.

Wesentlich ist, daß die Watte und der vliesartige Mantel vor der Benutzung des Tampons 6 weitgehend trocken sind und bis zur Benutzung auch trocken gehalten werden, so daß das Bakterienlyophilisat im unbenutzten Zustand auch bei längerer Aufbewahrungszeit nicht aktiviert wird.

Um eine ausreichende Haltbarkeit und Wirksamkeit des Tampons im unbenutzten Zustand sichern zu können, wird der verpreßte Tampon luft- und feuchtigkeitsdicht verpackt

Das Bakterienlyophilisat mit dem Nährmedium wird auf das flache Vliesmaterial aufgestreut. Damit eine ausreichende Haftung auf dem Vliesmaterial während der Ummantelung des locker aufgerollten Wattebahnabschnittes erreicht wird, kann das Vliesmaterial in herkömmlicher Weise elektrostatisch leicht aufgeladen werden. Das Bakterienlyophilisat kann auch mit dem Vliesmaterial vor der Ummantelung verpreßt werden, wobei die Körnung und die Korngrößen dess Bakterienlyophilisates von Bedeutung sein können. Das gleiche gilt auch für das pulverförmige Nährmedium, das gleichzeitig mit dem Bakterienlyophilisat auf das Vliesmaterial aufgebracht wird. Dabei kann das Nährmedium auch nach dem Gesichtspunkt besonders ausgewählt sein, daß es zusammen mit dem Lyophilisat gut auf dem Vliesmaterial haftet und klebrige Eigenschaften aufweist, die einer Aktivierung der Bakterien durch Feuchtigkeitszutritt im Anwendungsfall eines erfindungsgemäßen Tampons nicht hinderlich sind.

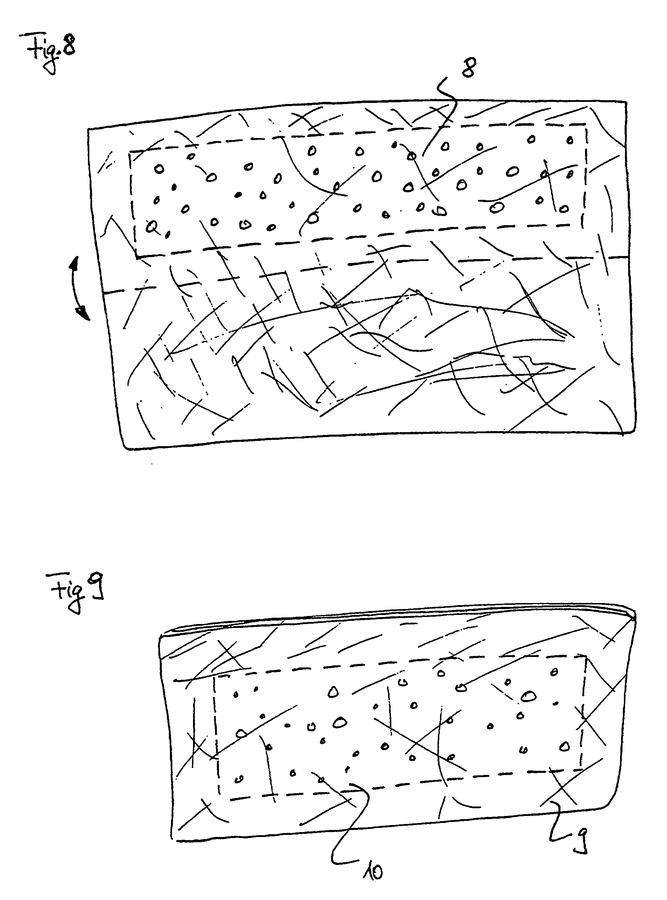

Nach einer Variante besitzt der vliesartige Stoff eine doppelte Breite. Das Bakterienlyophilisat mit dem Nährmedium wird auf die eine Hälfte 8 des flachen Vliesmaterial aufgetragen. Anschließend wird das Vliesmaterial in Längsrichtung gefaltet (Fig. 8) und an seinen übereinanderliegenden Rändern 9 dicht verpreßt (Fig. 9), so daß das Bakterienlyophilisat mit seinem Nährmedium zwischen den Vliesmaterialhälften eingeschlossen ist. Das gefaltete Vliesmaterial dient zur Ummantelung des lose aufgerollten Watteabschnittes (Fig. 3). Anschließend wird der so vorbereitete Tampon in die in Fig. 7 gezeigte gebrauchsfertige Form radial zusammengepreßt

Die Erfindung ist nicht auf die beschriebenen Ausführungsbeispiele beschränkt. So kann es vorteilhaft sein, ein zu einer Rolle aufgewickeltes doppellagiges Vliesband mit aufeinanderfolgenden, an ihren Rändern verpreßten Abschnitten herzustellen, in denen sich jeweils das Bakterienlyophilisat mit dem Nährmedium befindet, wobei zur Ummantelung eines zunächst nur locker aufgerollten Wattebahnabschnittes ein doppelwandiger Mantelabschnitt von der Rolle abgetrennt wird.

Die Erfindung ist nicht auf die Verwendung eines einzelnen Bakterienstammes und die hieran angepaßten Nährmedien beschränkt. So können auch probiotische Bakteriengemische verwendet werden. Je nach dem Anwendungsort des Tampons sind die zur Symbiontentherapie notwendigen Bakterien mit ihren Nährmedien in ausreichender Konzentration besonders ausgewählt.

Der Feuchtigkeitsgehalt der weitgehend trockenen erfindungsgemäßen Tampons sollte 5% nicht überschreiben. Die Menge des im Tampon enthaltenen Bakterienlyophilisates zusammen mit dem Nährmedium hängt von seiner therapeutischen Anwendung ab.

## Patentansprüche

1. Tampon zur Anwendung in Körperhöhlen aus einem aufgerollten plissierten Wattebahnabschnitt, der von einem vliesartigen Stoffabschnitt ummantelt und in eine anwendungsgerechte stäbchenförmige Gestalt radial verpreßt ist, **dadurch gekennzeichnet, daß** der Wattebahnabschnitt aus einem hydrophoben oder weitgehend hydrophoben Material besteht und zwischen dem vilesartigen Stoffabschnitt und dem aufgerollten Wattebahnabschnitt ein Bakterienlyophilisat mit zugehörigem Nährmedium eingebracht ist, wobei der Tampon vor seiner Benutzung weitgehend trocken gehalten und vor Feuchtigkeitszutritt geschützt ist.

2. Tampon nach Anspruch 1, **dadurch gekennzeichnet, daß** das Lyophilisat mit dem Nährmedium in pulvriger oder körniger Form auf den vliesartigen Stoffabschnitt als Träger aufgebracht ist.

3. Tampon nach Anspruch 2, **dadurch gekennzeichnet, daß** der vliesartige Stoff doppelwandig mit eingeschlossenem Lyophilisat und Nährmedium ausgebildet ist.

4. Tampon nach Anspruch 2, **dadurch gekennzeichnet, daß** der vliesartige Stoff zur Haftung des Lyophilisates mit dem Nährmedium auf dem vliesartigen Stoff elektrostatisch aufgeladen ist.

5. Tampon nach einem der vorstehenden Ansprüche,
**dadurch gekennzeichnet, daß**
das Lyophilisat globulinartig ausgebildet ist.

6. Tampon nach einem der vorstehenden Ansprüche,
**dadurch gekennzeichnet, daß** das pulverförmige Nährmedium ausgewählte klebrige Eigenschaft aufweist, die seine Haftung zusammen mit dem Lyophilisat auf dem vliesartigen Stoff ermöglichen oder begünstigen.

7. Tampon nach einem der vorstehenden Ansprüche,
**dadurch gekennzeichnet, daß**
sein Feuchtigkeitsgehalt vor der Benutzung 5 Gew.-% nicht überschreitet und vorzugsweise unter 5 Gew.-% betragt.

## Claims

1. Tampon used in visceral cavities made of a rolled up pleated cotton wool tape partition being coated by a fleecelike cloth partition and pressed to assume an easily applicable stick-shaped form **characterised in that** the cotton wool tape partition consists of a hydrophobic or mainly hydrophobic material and that a bacteria lyophilisate with an appropriate culture medium is inserted between the fleecelike partition and the rolled up cotton wool tape partition, wherein the tampon is kept mainly dry before use and is protected against access of moisture.

2. Tampon according to claim 1 **characterised in that** the lyophilisate is applied together with the culture medium in powdery or grainy form on the fleecelike cloth partition as a carrier.

3. Tampon according to claim 2 **characterised in that** the fleecelike cloth has a double lining provided with enclosed lyophilisate and culture medium.

4. Tampon according to claim 2 **characterised in that** the fleecelike cloth is charged electrostatically to provide adhesion of the lyophilisate with the culture medium on the fleecelike cloth.

5. Tampon according to one of the aforementioned claims **characterised in that** the lyophilisate is in globulin form.

6. Tampon according to one of the aforementioned claims **characterised in that** the culture medium in a powdery form comprises predetermined adhesive features allowing or fostering its adhesion together with the lyophilisate on the fleecelike cloth.

7. Tampon according to one of the aforementioned claims **characterised in that** its moisture content before use does not exceed 5 percent by weight and preferably remains below 5 percent by weight.

## Revendications

1. Tampon utilisé dans des cavités du corps fabriqué par un panneau de ouate plissé et enroulé, qui est enveloppé par un panneau d'étoffe semblable à la toison et qui est pressé dans une forme facile à utiliser rassemblant à un bâton, **caractérisé par le fait que** le panneau de ouate consiste dans un matériel hydrophobe ou d'un très haut degré hydrophobe, et qu'entre le panneau d'étoffe semblable à la toison et le panneau de ouate plissé et enroulé soit placé un lyophilisante bactérien, qui soit muni d'un milieu de culture approprié, qui serve à tenir le tampon sec à un haut degré et qui évite l'accès d'humidité avant l'usage.

2. Tampon comme celui défini dans la revendication 1 **caractérisé par le fait que** la lyophilisante soit appliqué avec le milieu de culture sous forme pulvérisée ou granulée sur l'étoffé semblable à la toison en tant que matériel porteur.

3. Tampon comme celui défini dans la revendication 2 **caractérisé par le fait que** l'étoffe semblable à la toison soit formé à double cloison qui soit muni d'une lyophilisante enveloppée et d'un milieu de culture.

4. Tampon comme celui défini dans la revendication 2 **caractérisé par le fait que** l'étoffe semblable à la toison soit chargé de manière électrostatique pour garantir l'adhésion de la lyophilisante avec le milieu de culture sur l'étoffe semblable à la toison.

5. Tampon comme celui défini dans les revendications précédentes, **caractérisé par le fait que** la lyophilisante soit disposée sous forme globulaire.

6. Tampon comme celui défini dans les revendications précédentes, **caractérisé par le fait que** le milieu de culture pulvérisé ait une qualité prédéterminée adhésive, qui garantie ou favorise son adhésion avec la tyophitisante sur l'étoffe semblable à la toison.

7. Tampon comme celui défini dans les revendications précédentes, **caractérisé par le fait que** son degré d'humidité avant l'usage ne dépasse pas 5% de poids, et que ledit degré s'élève de préférence à moins de 5% de poids.
